# EUROPEAN PATENT APPLICATION

(11) **EP 4 772 170 A1**
(43) Date of publication of application: **08.07.2026**
(21) Application number: 24862119.5
(22) Date of filing: 09.09.2024
(51) Int. Cl.: A61K 9/10, A61K 9/08, A61K 9/06, A61K 31/496, A61P 25/24, A61P 25/18, A61P 25/14, A61P 25/28

(54) **BREXPIPRAZOLE GEL FOR INJECTION**

(30) Priority: 08.09.2023 CN 202311156607
(71) Applicant: Bostal Drug Delivery Co., Ltd., Guangzhou, Guangdong 510530 (CN); Bostal LLC, Edison, New Jersey 08837 (US)
(72) Inventor: LIU, Rong, Edison New Jersey 08837 (US); WU, Shaoxian, Guangzhou, Guangdong 510530 (CN); YUE, Zhanguo, Guangzhou, Guangdong 510530 (CN); FENG, Sixin, Guangzhou, Guangdong 510530 (CN); QI, Xiaohong, Edison New Jersey 08837 (US); LI, Wei, Guangzhou, Guangdong 510530 (CN)
(74) Representative: Lavoix
(86) International application number: PCT/CN2024/117842
(87) International publication number: WO 2025/051281

(57) **Abstract**

A brexpiprazole gel, comprising brexpiprazole or a pharmaceutically acceptable salt thereof, at least one biocompatible biodegradable sustained-release material and at least one solvent, wherein the sum of the content of the sustained-release material and three times the content of brexpiprazole or the pharmaceutically acceptable salt thereof is not higher than 500 mg/g, calculated on the basis of the total mass of brexpiprazole or the pharmaceutically acceptable salt thereof, the sustained-release material and the solvent.

## Description

### CROSS-REFERENCE TO RELATED APPLICATIONS

The present application claims priority to Chinese Patent Application No. 202311156607.4, filed with the China National Intellectual Property Administration on September 8, 2023, entitled "GEL", the entire contents of which are incorporated herein by reference.

### TECHNICAL FIELD

The present disclosure generally relates to the pharmaceutical field, and specifically to a gel dosage form of brexpiprazole for injection.

### BACKGROUND

Schizophrenia is a serious mental disorder that often manifests clinically as a syndrome with varying symptoms, involving disturbances in perception, thinking, emotion, behavior, and other aspects, as well as disorganization of mental activity. The course of the disease is prolonged, with recurrent episodes or exacerbations, and some patients eventually experience deterioration and mental disability.

Brexpiprazole, as an agonist of 5-HT1A receptor and dopamine D2 receptor and an antagonist of 5-HT2A receptor, is clinically used to treat schizophrenia, major depressive disorder, and agitation associated with dementia due to Alzheimer's disease. It has better efficacy and tolerability and can reduce adverse reactions such as akathisia, restlessness, or insomnia in patients. Currently, for the treatment of patients with mental illness, oral administration of brexpiprazole shows poor compliance and missed doses, leading to a high relapse rate of the disease. Therefore, there is a need to develop long-acting brexpiprazole formulations to address these issues for patients.

### SUMMARY OF THE INVENTION

The object of the present invention is to develop a gel dosage form of brexpiprazole for easy injection administration. However, studies have found that gel dosage form of brexpiprazole is prone to crystallization during preparation or storage, especially the gel dosage form of brexpiprazole with high-concentration. How to reduce crystallization and enable gel dosage form of brexpiprazole to be administered in the form of a clear solution is an urgent problem to be solved in the development of gel dosage form of brexpiprazole. On the basis of this, enabling the sustained release of brexpiprazole at an appropriate rate after administration is another problem that urgently needs to be solved in the development of gel dosage form of brexpiprazole. One of the technical problems solved by the present invention is reducing crystallization, enabling the gel dosage form of brexpiprazole to be injected as a clear solution. A further technical problem solved by the present invention is to enable the sustained release of brexpiprazole at an appropriate rate after administration.

In one aspect, this disclosure relates to a gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

In another aspect, this disclosure relates to a method for use as an adjunctive therapy to the treatment of major depressive disorder in adults, comprising administering to a patient in need of the method an effective amount of the gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

In yet another aspect, this disclosure relates to a method for treating schizophrenia, comprising administering to a patient in need of the method an effective amount of the gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

In another aspect, this disclosure relates to a method for treating agitation associated with dementia due to Alzheimer's disease, comprising administering to a patient in need of the method an effective amount of the gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

In another aspect, this disclosure also provides a gel dosage form of brexpiprazole, comprising brexpiprazole or a pharmaceutically acceptable salt thereof, at least one biocompatible and biodegradable sustained-release material, and at least one solvent, wherein, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not more than 500 mg/g. Within this range, the gel dosage form of brexpiprazole can be a clear solution at room temperature, or a clear solution when the temperature is increased from room temperature to 60°C. Optionally, the sum of three times the content of the brexpiprazole or the pharmaceutically acceptable salt thereof and the content of the sustained-release material is not more than 455 mg/g, and within this range, the gel dosage form of brexpiprazole can be a clear solution at room temperature or at a temperature ranging from room temperature to 40°C. Optionally, the sum of three times the content of the brexpiprazole or the pharmaceutically acceptable salt thereof and the content of the sustained-release material is not more than 441 mg/g, and within this range, the gel dosage form of brexpiprazole can be a clear solution at room temperature or at a temperature ranging from room temperature to 35°C. Optionally, the sum of three times the content of the brexpiprazole or the pharmaceutically acceptable salt thereof and the content of the sustained-release material is not more than 400 mg/g, and within this range, the gel dosage form of brexpiprazole is a clear solution at room temperature.

In some embodiments, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not more than 499 mg/g, 498 mg/g, 497 mg/g, 496 mg/g, 495 mg/g, 494 mg/g, 493 mg/g, 492 mg/g, 491 mg/g, 490 mg/g, 489 mg/g, 488 mg/g, 487 mg/g, 486 mg/g, 485 mg/g, 484 mg/g, 483 mg/g, 482 mg/g, 481 mg/g, 480 mg/g, 479 mg/g, 478 mg/g, 477 mg/g, 476 mg/g, 475 mg/g, 474 mg/g, 473 mg/g, 472 mg/g, 471 mg/g, 470 mg/g, 469 mg/g, 468 mg/g, 467 mg/g, 466 mg/g, 465 mg/g, 464 mg/g, 463 mg/g, 462 mg/g, 461 mg/g, 460 mg/g, 459 mg/g, 458 mg/g, 457 mg/g, 456 mg/g, 455 mg/g, 454 mg/g, 453 mg/g, 452 mg/g, 451 mg /g, 450 mg/g, 449 mg/g, 448 mg/g, 447 mg/g, 446 mg/g, 445 mg/g, 444 mg/g, 443 mg/g, 442 mg/g, 441 mg/g, 440 mg/g, 439 mg/g, 438 mg/g, 437 mg/g, 436 mg/g, 435 mg/g, 434 mg/g, 433 mg/g, 432 mg/g, 431 mg/g, 430 mg/g, 429 mg/g, 428 mg/g, 427 mg/g, 426 mg/g, 425 mg/g, 424 mg/g, 423 mg/g, 422 mg/g, 421 mg/g, 420 mg/g, 419 mg/g, 418 mg/g, 417 mg/g, 416 mg/g, 415 mg/g, 414 mg/g, 413 mg/g, 412 mg/g, 411 mg/g, 410 mg/g, 409 mg/g, 408 mg/g, 407 mg/ g, 406 mg/g, 405 mg/g, 404 mg/g, 403 mg/g, 402 mg/g, 401 mg/g, 400 mg/g, 399 mg/g, 398 mg/g, 397 mg/g, 396 mg/g, 395 mg/g, 394 mg/g, 393 mg/g, 392 mg/g, 391 mg/g, 390 mg/g, 389 mg/g, 388 mg/g, 387 mg/g, 386 mg/g, 385 mg/g, 384 mg/g, 383 mg/g or 380 mg/g.

In some embodiments, the content of the brexpiprazole or the pharmaceutically acceptable salt thereof is not less than 28 mg/g. Optionally, the content of the brexpiprazole or the pharmaceutically acceptable salt thereof ranges from 28 mg/g to 102 mg/g, and further optionally from 66 mg/g to 73 mg/g.

In some embodiments, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not less than 322 mg/g. Optionally, the sum of three times the content of the brexpiprazole or the pharmaceutically acceptable salt thereof and the content of the sustained-release material ranges from 322 mg/g to 500 mg/g, optionally from 322 mg/g to 455 mg/g, optionally from 322 mg/g to 441 mg/g, or optionally from 322 mg/g to 400 mg/g.

In some embodiments, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not less than 323 mg/g, 324 mg/g, 325 mg/g, 326 mg/g, 327 mg/g, 328 mg/g, 329 mg/g, 330 mg/g, 331 mg/g, 332 mg/g, 333 mg/g, 334 mg/g, 335 mg/g, 336 mg/g, 337 mg/g, 338 mg/g, 339 mg/g, 340 mg/g, 341 mg/g, 342 mg/g, 343 mg/g, 344 mg/g, 345 mg/g, 346 mg/g, 347 mg/g, 348 mg/g, 349 mg/g, 350 mg/g, 351 mg/g, 352 mg/g, 353 mg/g, 354 mg/g, 355 mg/g, 356 mg /g, 357 mg/g, 358 mg/g, 359 mg/g, 360 mg/g, 361 mg/g, 362 mg/g, 363 mg/g, 364 mg/g, 365 mg/g, 366 mg/g, 367 mg/g, 368 mg/g, 369 mg/g or 370 mg/g.

In some embodiments, the sum of three times the content of the brexpiprazole or the pharmaceutically acceptable salt thereof and the content of the sustained-release material is in a range from 361 mg/g to 391 mg/g, optionally from 371 mg/g to 391 mg/g, and further optionally from 371 mg/g to 388 mg/g. The inventors unexpectedly discovered that, within these ranges, the described gel dosage form of brexpiprazole exhibits a satisfactory pore size distribution and sustained-release effect after injection.

In some embodiments, based on the total mass of the brexpiprazole or the pharmaceutically acceptable salt thereof, the sustained-release material and the solvent, the ratio of the content of the sustained-release material to the content of the brexpiprazole or the pharmaceutically acceptable salt thereof is (0.8-10):1, preferably (0.8-3.3):1, and more preferably (2.0-2.3):1. The inventors unexpectedly discovered that, based on controlling the range of the sum of three times the content of the brexpiprazole or the pharmaceutically acceptable salt thereof and the content of the sustained-release material, and by further controlling the range of a ratio of the content of the sustained-release material to the content of the brexpiprazole or the pharmaceutically acceptable salt thereof, the gel dosage form of brexpiprazole could achieve a more satisfactory pore size distribution and sustained-release effect after injection.

In another aspect, this disclosure also provides use of the gel dosage form of brexpiprazole in the manufacture of a medicine for use as an adjunctive therapy to the treatment of major depressive disorder in adults.

In another aspect, this disclosure also provides use of the gel dosage form of brexpiprazole in the manufacture of a medicine for treating schizophrenia.

In another aspect, this disclosure also provides use of the gel dosage form of brexpiprazole in the manufacture of a medicine for treating agitation associated with dementia due to Alzheimer's disease.

In another aspect, this disclosure also provides a method for use as an adjunctive therapy to the treatment of major depressive disorder in adults, comprising administering to a patient in need of the method an effective amount of the gel dosage form provided by the present disclosure.

In yet another aspect, this disclosure also provides a method for treating schizophrenia, comprising administering to a patient in need of the method an effective amount of the gel dosage form provided by this disclosure.

In another aspect, this disclosure also provides a method for treating agitation associated with dementia due to Alzheimer's disease, comprising administering to a patient in need of the method an effective amount of the gel dosage form provided by this disclosure.

### Description

The following description includes specific details to provide a comprehensive understanding of the various disclosed embodiments. However, those skilled in the art will recognize that the embodiment can still be implemented by using other methods, components, materials and the like, without employing one or more of these specific details.

Unless otherwise required in the present application, throughout the specification and the appended claims, the words "comprising", "including", "containing", and "having" shall be interpreted in an open-ended, inclusive sense, meaning "including but not limited to".

When used in this disclosure and the appended claims, a singular designation without a quantity indication includes a plural designation unless the context clearly specifies otherwise.

Throughout this specification, the terms "an embodiment", "embodiments", "in another embodiment", or "in some embodiments" mean that at least one embodiment includes a specific reference element, structure, or feature related to that embodiment. Therefore, the phrases "in one embodiment", "in an embodiment", "in another embodiment", or "in some embodiments" that appear in different places throughout the specification do not necessarily all refer to the same embodiment. Furthermore, specific elements, structures, or features may be combined in one or more embodiments in any suitable manner.

It should be understood that the singular articles "a", "an" and "the" used in this disclosure and the appended claims include plural objects unless otherwise expressly stated in the text. Therefore, for example, the sustained-release tablets containing "pharmaceutically acceptable excipients" include one pharmaceutically acceptable excipient, or two or more pharmaceutically acceptable excipients.

### Definition

In this disclosure, the term "brexpiprazole" refers to 7-{4-[4-(1-benzothiophen-4-yl)piperazin-1-yl]butoxy}quinolin-2(1H)-one, with the English name brexpiprazole.

In this disclosure, the term "acceptable salt" refers to an acid addition salt or base addition salt prepared from a pharmaceutically acceptable acid or base; acceptable acid addition salts include, but are not limited to, benzenesulfonic acid, acetic acid, propionic acid, oxalic acid, isobutyric acid, maleic acid, malonic acid, benzoic acid, trans-butenedioic acid, succinic acid, suberic acid, lactic acid, mandelic acid, gluconic acid, phthalic acid, p-toluenesulfonic acid, citric acid, salicylic acid, tartaric acid, methanesulfonic acid, isonicotinic acid, acid citrate, oleic acid, tannic acid, pantothenic acid, hydrogen tartrate, ascorbic acid, gentian acid, fumaric acid, saccharic acid, formic acid, ethanesulfonic acid, amino acids, hydrochloric acid, hydrobromic acid, hydroiodic acid, nitric acid, carbonic acid, phosphoric acid, phosphorous acid, sulfuric acid and the like; acceptable base addition salts include, but are not limited to, diethanolamine salts, calcium salts, ammonium salts, lithium salts, sodium salts, potassium salts, aluminum salts, magnesium salts, zinc salts, bismuth salts and the like.

In this disclosure, the term "biocompatible and biodegradable sustained-release material" refers to a natural or synthetic biomedical material that is biocompatible and acceptable, can regulate the medicine release rate, and is continuously degraded under the action of body fluids, absorbed or excreted by the body, and eventually completely replaced by new tissue.

In this disclosure, the term "gel dosage form" means, in situ gel dosage form, also known as injectable gel dosage form, which is usually composed of a biocompatible solvent and a biodegradable polymer; after being injected in a liquid state, it undergoes a rapid phase transition at the administration site, transforming from a liquid state to a solid or semi-solid state, thereby prolonging the residence time of the medicine at the administration site. Meanwhile, as the biodegradable polymer slowly degrades, the encapsulated medicine is fully released within weeks to months, thus achieving the purpose of sustained and controlled release.

In this disclosure, the term "room temperature" refers to 10°C to 30°C.

In the present invention, the term "clear" means that the gel dosage form contains no precipitate or deposit visible to the naked eye.

In the present invention, the term "suspension" means the gel dosage form contains precipitate or deposit visible to the naked eye.

### BRIEF DESCRIPTION OF THE DRAWINGS

FIG. 1 is a relationship diagram showing the effect of three times the concentration of API and the PLGA concentration on crystallization.
FIG. 2a shows the pore size distribution test results (0-1000 nm) after gel dosage form solidification.
FIG. 2b shows the pore size distribution test results (0-10000 nm) after gel dosage form solidification.
FIG. 3a shows a comparison of rat pharmacokinetic (PK) experiments with different formulations of brexpiprazole long-acting gel dosage form.
FIG. 3b is a partially enlarged view of FIG. 3a.

### DETAILED DESCRIPTION

In one aspect, this disclosure relates to a gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

In some embodiments, the concentration of the brexpiprazole or the pharmaceutically acceptable salt thereof in the gel dosage form of brexpiprazole of this disclosure is not less than 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL or 130 mg/mL.

In some embodiments, the sum of three times the concentration of the brexpiprazole or the pharmaceutically acceptable salt thereof and the concentration of the sustained-release material in the gel dosage form of brexpiprazole of this disclosure is not more than 600 mg/mL, 595 mg/mL, 590 mg/mL, 585 mg/mL, 580 mg/mL, or 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL, or 490 mg/mL.

In some embodiments, exemplary examples of biocompatible and biodegradable sustained-release materials that can be used to form the gel dosage form of this disclosure include, but are not limited to, lactide-glycolide copolymers with a molar ratio of lactic acid (lactide) to glycolic acid (glycolide) of about 50:50 to about 95:5. In the present application, lactide-glycolide copolymer and poly(lactic-co-glycolic acid) are the same substance, abbreviated as PLGA.

In some embodiments, exemplary examples of at least one solvent that can be used in the gel dosage form of this disclosure include, but are not limited to, organic solvents.

In some embodiments, exemplary examples of organic solvents that can be used in this disclosure include, but are not limited to, alcohol solvents, ester solvents, ether solvents, ketone solvents, and amide solvents.

In some embodiments, exemplary examples of alcohol solvents that can be used in this disclosure include, but are not limited to, ethanol, n-propanol, isopropanol, n-butanol, propylene glycol, glycerol, or 1,3-butanediol.

In some embodiments, exemplary examples of ester solvents that can be used in this disclosure include, but are not limited to, ethyl acetate, methyl acetate, ethyl formate, butyl acetate, diethyl malonate, n-butyl acetate, or isobutyl acetate.

In some embodiments, exemplary examples of ether solvents that can be used in this disclosure include, but are not limited to, tetrahydrofuran, methyltetrahydrofuran, diethylene glycol monomethyl ether, or tetrahydrofurfuryl alcohol polyethylene glycol ether.

In some embodiments, exemplary examples of ketone solvents that can be used in this disclosure include, but are not limited to, acetone, methyl ethyl ketone, diethyl ketone, or methyl isobutyl ketone.

In some embodiments, exemplary examples of amide solvents that can be used in this disclosure include, but are not limited to, formamide, dimethylformamide, dimethylacetamide, hexamethylphosphoramide, N-methyl-2-pyrrolidone, N-cyclohexyl-2-pyrrolidone, N-hydroxyethyl-2-pyrrolidone, 2-pyrrolidone, or N-ethyl-2-pyrrolidone.

In another aspect, this disclosure relates to a method for use as an adjunctive therapy to the treatment of major depressive disorder in adults, comprising administering to a patient in need of the method an effective amount of a gel dosage form of brexpiprazole comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL or 130 mg/mL.

In some embodiments, according to the gel dosage form that can be used in the administration method of this disclosure, the sum of three times the concentration of the brexpiprazole or the pharmaceutically acceptable salt thereof and the concentration of the sustained-release material is not more than 600 mg/mL, 595 mg/mL, 590 mg/mL, 585 mg/mL, 580 mg/mL, or 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL or 490 mg/mL.

In yet another aspect, this disclosure relates to a method for treating schizophrenia, comprising administering to a patient in need of the method an effective amount of a gel dosage form of brexpiprazole comprising brexpiprazole or a pharmaceutically acceptable salt thereof, at least one biocompatible and biodegradable sustained-release material, and at least one solvent.

In some embodiments, the gel dosage form of brexpiprazole that can be used in the administration method of this disclosure has a brexpiprazole or a pharmaceutically acceptable salt thereof at a concentration of not less than 80 mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL or 130 mg/mL.

In some embodiments, according to the gel dosage form of brexpiprazole that can be used in the administration method of this disclosure, the sum of three times the concentration of the brexpiprazole or the pharmaceutically acceptable salt thereof and the concentration of the sustained-release material is not more than 600 mg/mL, 595 mg/mL, 590 mg/mL, 585 mg/mL, 580 mg/mL or 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL or 490 mg/mL.

In another aspect, this disclosure relates to a method for treating agitation associated with dementia due to Alzheimer's disease, comprising administering to a patient in need of the method an effective amount of the gel dosage form of brexpiprazole comprising brexpiprazole or a pharmaceutically acceptable salt thereof, at least one biocompatible and biodegradable sustained-release material, and at least one solvent.

In some embodiments, the gel dosage form of brexpiprazole that can be used in the administration method of this disclosure has a brexpiprazole or a pharmaceutically acceptable salt thereof at a concentration of not less than 80 mg/mL, 81 mg/mL, 82 mg/mL, 83 mg/mL, 84 mg/mL, 85 mg/mL, 85 mg/mL, 86 mg/mL, 87 mg/mL, 88 mg/mL, 89 mg/mL, 90 mg/mL, 91 mg/mL, 92 mg/mL, 93 mg/mL, 94 mg/mL, 95 mg/mL, 96 mg/mL, 97 mg/mL, 98 mg/mL, 99 mg/mL, 100 mg/mL, 101 mg/mL, 102 mg/mL, 103 mg/mL, 104 mg/mL, 105 mg/mL, 106 mg/mL, 107 mg/mL, 108 mg/mL, 109 mg/mL, 110 mg/mL, 111 mg/mL, 112 mg/mL, 113 mg/mL, 114 mg/mL, 115 mg/mL, 116 mg/mL, 117 mg/mL, 118 mg/mL, 119 mg/mL, 120 mg/mL, 121 mg/mL, 122 mg/mL, 123 mg/mL, 124 mg/mL, 125 mg/mL, 126 mg/mL, 127 mg/mL, 128 mg/mL, 129 mg/mL or 130 mg/mL.

In some embodiments, according to the gel dosage form of brexpiprazole that can be used in the administration method of this disclosure, the sum of three times the concentration of the brexpiprazole or the pharmaceutically acceptable salt thereof and the concentration of the sustained-release material is not more than 600 mg/mL, 595 mg/mL, 590 mg/mL, 585 mg/mL, 580 mg/mL or 575 mg/mL, 570 mg/mL, 570 mg/mL, 565 mg/mL, 560 mg/mL, 555 mg/mL, 550 mg/mL, 545 mg/mL, 540 mg/mL, 535 mg/mL, 530 mg/mL, 525 mg/mL, 520 mg/mL, 515 mg/mL, 510 mg/mL, 505 mg/mL, 500 mg/mL, 495 mg/mL or 490 mg/mL.

In some embodiments, the gel dosage form of the present disclosure provides a release duration of at least about 1 month for brexpiprazole or a pharmaceutically acceptable salt thereof.

The gel dosage form of the present invention can be prepared using conventional preparation methods, for example, by the following method: adding the formulation amount of N-methyl-2-pyrrolidone to a container containing the formulation amount of brexpiprazole, shaking manually to completely dissolve the brexpiprazole, then adding the formulation amount of poly(lactic-co-glycolic acid) to the container, sealing it, and placing it on a roller mixer to mix until dissolved or evenly suspended to obtain a gel dosage form.

The containers can be conventional ones, such as vials, screw-neck glass bottles, blue-cap bottles, polytetrafluoroethylene (PTFE) bottles, polypropylene bottles and the like.

In the following sections, this disclosure will be explained in detail through the following embodiments in order to better understand the various aspects and advantages of this disclosure. However, it should be understood that the following embodiments are non-limiting and are only used to illustrate certain implementations of this disclosure.

The reagents and equipment used in the embodiments of this disclosure are all conventional and commercially available.

### Example 1

### Preparation of gel dosage form of brexpiprazole

In this example, the formulation of the gel dosage form of brexpiprazole is shown in Table 1, where API is brexpiprazole, PLGA is poly(lactic-co-glycolic acid) 50:50, and NMP is N-methyl-2-pyrrolidone. The specific preparation method of this formulation gel dosage form is as follows:
The formulation amount of N-methyl-2-pyrrolidone was added to a 10 ml vial containing the formulation amount of brexpiprazole. It was shaken manually for 1-2 minutes to completely dissolve the brexpiprazole. Then, the formulation amount of poly(lactic-co-glycolic acid) was added to the container. The container was sealed and placed on a roller mixer (manufacturer: CRYSTAL, model: MR-020) to mix at a speed of 20RPM until the poly(lactic-co-glycolic acid) was uniformly suspended, thereby obtaining a gel dosage form. It was visually observed as a suspension at room temperature (20-30°C).

### Examples 2 to 21

The preparation methods of Examples 2 to 21 are the same as those of Example 1, except that the composition of the formulations are different. The specific formulation details are shown in Table 1. The APIs are all brexpiprazole, PLGA is poly(lactic-co-glycolic acid) 50:50, and NMP is N-methyl-2-pyrrolidone. In Example 4, the PLGA used is poly(lactic-co-glycolic acid) 50:50, 0.10-0.30 dL/g, commonly referred to as 0.15 dL/g, and manufactured by Ashland; in Example 19, the PLGA is 5050, 0.20-0.40 dL/g, commonly referred to as 0.28 dL/g, and manufactured by Ashland; in the other examples, the PLGA is poly(lactic-co-glycolic acid) 50:50, 0.16-0.24 dL/g, commonly referred to as 0.21 dL/g, and manufactured by Evonik. The gel dosage forms prepared in Examples 2 to 7 are suspensions at room temperature (20-30°C), and the gel dosage forms prepared in Examples 8 to 21 are clear solutions at room temperature (20-30°C).

**Table 1**

| Example | Amounts of Each Component | | | API Content mg/g | PLGA Content mg/g | API Content *3 +PLGA Content mg/g |
|---|---|---|---|---|---|---|
| | API /mg | PLGA /mg | NMP /mg | | | |
| 1 | 120 | 360 | 960 | 83.3 | 250.0 | 500.0 |
| 2 | 120 | 300 | 960 | 87.0 | 217.4 | 478.3 |
| 3 | 120 | 240 | 960 | 90.9 | 181.8 | 454.5 |
| 4 | 60 | 305 | 735 | 54.5 | 277.3 | 440.9 |
| 5 | 120 | 180 | 960 | 95.2 | 142.9 | 428.6 |
| 6 | 120 | 300 | 1160 | 75.9 | 189.9 | 417.7 |
| 7 | 120 | 200 | 1060 | 87.0 | 144.9 | 405.8 |
| 8 | 60 | 300 | 840 | 50.0 | 250.0 | 400.0 |
| 9 | 240 | 560 | 2480 | 73.2 | 170.7 | 390.2 |
| 10 | 240 | 200 | 1920 | 101.7 | 84.7 | 389.8 |
| 11 | 150 | 500 | 1800 | 61.2 | 204.1 | 387.8 |
| 12 | 50 | 500 | 1200 | 28.6 | 285.7 | 371.4 |
| 13 | 200 | 500 | 2300 | 66.7 | 166.7 | 366.7 |
| 14 | 100 | 500 | 1600 | 45.5 | 227.3 | 363.6 |
| 15 | 360 | 720 | 3900 | 72.3 | 144.6 | 361.4 |
| 16 | 150 | 500 | 2000 | 56.6 | 188.7 | 358.5 |
| 17 | 240 | 560 | 2800 | 66.7 | 155.6 | 355.6 |
| 18 | 200 | 500 | 2400 | 64.5 | 161.3 | 354.8 |
| 19 | 240 | 560 | 3000 | 63.2 | 147.4 | 336.8 |
| 20 | 60 | 140 | 750 | 63.2 | 147.4 | 336.8 |
| 21 | 300 | 600 | 3750 | 64.5 | 129.0 | 322.6 |

In the table, API content = mass of API / (mass of API + mass of PLGA + mass of NMP) * 100%; PLGA content = mass of PLGA / (mass of API + mass of PLGA + mass of NMP) * 100%; API content * 3 + PLGA content = 3 * mass of API / (mass of API + mass of PLGA + mass of NMP) * 100% + mass of PLGA / (mass of API + mass of PLGA + mass of NMP) * 100%.

### Experiment Example 1: Crystallization Experiment

### (1) Room temperature crystallization experiment

### Experimental methods and instruments:

1) Instrument: Clarity tester (Manufacturer: Jingtuo Instruments, Model: YB-IIA)
2) Experimental method: The freshly prepared gel dosage form samples from Examples 1 to 21 were placed at room temperature. The gel dosage form samples were then placed under a clarity tester and visually observed for crystal precipitation, and the crystallization time was recorded.

After preparation, it was observed at room temperature for 2 hours: the freshly prepared gel dosage form sample was placed at room temperature for 2 hours, and then the gel dosage form sample was placed under a clarity tester to visually observe whether crystals had precipitated.

After preparation, it was observed at room temperature for 24 hours: the freshly prepared gel dosage form sample was placed at room temperature for 24 hours, and then the gel dosage form sample was placed under a clarity tester to visually observe whether crystals had precipitated.

The experimental results of the room temperature crystallization experiment are shown in Table 2:

**Table 2**

| Example | After preparation, observe at room temperature for 2 hours | After preparation, observe at room temperature for 24 hours |
|---|---|---|
| 1 | Suspension | Suspension |
| 2 | Suspension | Suspension |
| 3 | Suspension | Suspension |
| 4 | Suspension | Suspension |
| 5 | Suspension | Suspension |
| 6 | Suspension | Suspension |
| 7 | Suspension | Suspension |
| 8 | Clear | Clear |
| 9 | Clear | Clear |
| 10 | Clear | Clear |
| 11 | Clear | Clear |
| 12 | Clear | Clear |
| 13 | Clear | Clear |
| 14 | Clear | Clear |
| 15 | Clear | Clear |
| 16 | Clear | Clear |
| 17 | Clear | Clear |
| 18 | Clear | Clear |
| 19 | Clear | Clear |
| 20 | Clear | Clear |
| 21 | Clear | Clear |

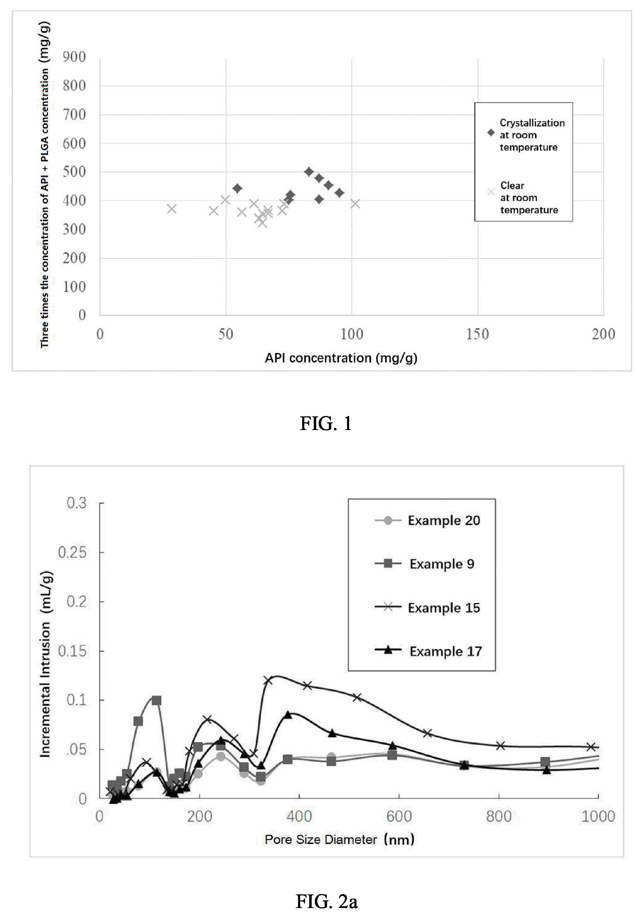

The relationship diagram illustrating the effect of three times the API content and the PLGA content on crystallization behavior was plotted, with API content (mg/g) as the x-axis and the sum of three times the API content and the PLGA content (mg/g) as the y-axis, as shown in FIG. 1.

### (2) Heating crystallization experiment

### Experimental methods and instruments:

1) Instruments: Heating magnetic stirrer (manufacturer: Gongyi Yuhua Instruments, model: DF·101S), clarity tester (manufacturer: Jingtuo Instruments, model: YB-IIA)
2) Experimental method: a stir bar was added to the gel-containing vial with precipitated crystals, the vial was placed in a heating magnetic stirrer, heated in a 35°C water bath while stirring for 30 minutes at a speed of 10 RPM. After 30 minutes, the sample was taken out and placed under a clarity tester to visually observe whether the crystals in the sample had completely dissolved. If dissolution was complete, the sample was removed and placed under a clarity tester at room temperature to observe the specific crystallization time visually. If dissolution was not complete, the sample was heated in a 40°C water bath while stirring for 30 minutes. After 30 minutes, the sample was removed and observed to check whether the crystals in the sample had completely dissolved. If dissolution was complete, the sample was removed and placed under a clarity tester at room temperature to observe the specific crystallization time visually. If complete dissolution was not achieved, the above steps were repeated, sequentially conducting water bath heating at 50°C and 60°C, and the specific crystallization time was recorded.

Heating to 35°C/heating time 30min: After heating the gel dosage form sample in a 35°C water bath for 30min, the sample was taken out and placed under a clarity tester to observe its appearance with the naked eye.

Heating to 40°C/heating time 30min: After heating the gel dosage form sample in a 40°C water bath for 30min, the sample was taken out and placed under a clarity tester to observe its appearance with the naked eye.

Heating to 50°C/heating time 30min: After heating the gel dosage form sample in a 50°C water bath for 30min, the sample was taken out and placed under a clarity tester to observe its appearance with the naked eye.

Heating to 60°C/heating time 30min: After heating the gel dosage form sample in a 60°C water bath for 30min, the sample was taken out and placed under a clarity tester to observe its appearance with the naked eye.

After heating and dissolving, it was observed at room temperature for 3 hours: the gel dosage form sample, which had cleared upon heating, was placed at room temperature for 3 hours; the sample was then taken out and placed under a clarity tester to observe whether crystals had precipitated.

After heating and dissolving, it was observed at room temperature for 3 days: the gel dosage form sample, which had cleared upon heating, was placed at room temperature for 3 days; the sample was then taken out and placed under a clarity tester to observe whether crystals had precipitated.

The experimental results of the heating crystallization experiment are shown in Table 3:

**Table 3**

| Example | After preparation, observe at room temperature for 2 hours | Heating to 35°C/heating time 30min | Heating to 40°C/heating time 30min | Heating to 50°C/heating time 30min | Heating to 60°C/heating time 30min | After heating and dissolving, observe at room temperature for 3 hours | After heating and dissolving, observe at room temperature for 3 days |
|---|---|---|---|---|---|---|---|
| 1 | Suspension | Largely Undissolved | Largely Undissolved | Undissolved | Dissolved and clear | Dissolved and clear | Crystallized |
| 2 | Suspension | Largely Undissolved | Undissolved | Undissolved | Dissolved and clear | Dissolved and clear | Crystallized |
| 3 | Suspension | Undissolved | Undissolved | Dissolved and clear | - | Dissolved and clear | Crystallized |
| 4 | Suspension | Dissolved and clear | Dissolved and clear | | - | Dissolved and clear | Crystallized |
| 5 | Suspension | Undissolved | Dissolved and clear | | - | Dissolved and clear | Crystallized |
| 6 | Suspension | Slightly Undissolved | Slightly Undissolved | Dissolved and clear | - | Dissolved and clear | Crystallized |
| 7 | Suspension | Dissolved and clear | - | - | - | Crystallized | Crystallized |

### Experimental Example 2: Pore Size Distribution Test

The gel dosage form of the present invention would solidify after injection to form a solid state. This section measured the pore size distribution inside the solid material formed after gel dosage form injection and solidification.

### Experimental methods and instruments:

### a. Preparation of gel dosage form samples

Fresh gel dosage form samples were prepared according to the component content ratios in Examples 9, 15, 17, and 20, using the same preparation method as in the above examples. The amounts of each component are as follows:
Corresponding to the proportions in Example 9, the specific amounts of each component are: API 240mg, PLGA 560mg, and NMP 2480mg;
Corresponding to the proportions in Example 15, the specific amounts of each component are: API 180mg, PLGA 360mg, and NMP 1950mg;
Corresponding to the proportions in Example 17, the specific amounts of each component are: API 240mg, PLGA 560mg, and NMP 2800mg;
Corresponding to the proportions in Example 20, the specific amounts of each component are: API 240mg, PLGA 560mg, and NMP 3000mg.

The gel dosage form samples were sterilized by 25 kGy Gamma irradiation. The final product was obtained after sterilization. Before use, the gel dosage form sample was drawn into a 1ml syringe and kept for later use.

### b. Subcutaneous injection in rats

Male SD rats (purchased from Zhuhai Bes Test Bio-Tech Co., Ltd.) weighing approximately 200-400g were selected. All rats were housed under the environment of 20-25°C and 40-70% relative humidity (RH), fed with standard feed (SPF rat and mouse maintenance feed, Shenyang Maohua Biotechnology Co., Ltd.), and allowed free access to food and water. Rats were randomly divided into four groups, corresponding to the gel dosage form administration groups of Examples 9, 15, 17, and 20 after the treatment in step a above, respectively. The administration method was as follows: The test rats were injected subcutaneously with gel dosage form of brexpiprazole. Each rat received one injection each on the left and right sides of the neck-shoulder region, with each injection corresponding to a dose of brexpiprazole of 15 mg, that is, the total dose received by each rat was 30 mg.

### c. Material Collection

Forty-eight hours after administration, the rats were sacrificed and the injection site was dissected. Complete solidified gel dosage form samples were taken out, and solidified gel dosage form samples with intact shape and no tissue adhesion were selected for subsequent freeze-drying process.

### d. Freeze-Drying

The extracted gel dosage form samples were placed in 10 mL borosilicate vials and lyophilized in a freeze dryer, sequentially undergoing a continuous process of pre-freezing, primary drying, and secondary drying. The freeze-drying equipment and parameters are shown in Table 4.

**Table 4**

| Freeze Dryer | | | | | | | | |
|---|---|---|---|---|---|---|---|---|
| Manufacturer | | SP scientific | | | | | | |
| Model | | Advantage Plus-EL-85 | | | | | | |
| Freeze-drying parameters | | | | | | | | |

| Pre-freezing | | | | | | | | |
|---|---|---|---|---|---|---|---|---|
| Temperature | -40°C | | Cooling time | | 60min | Holding time | | 240min |

| Primary drying | | | | | | | | |
|---|---|---|---|---|---|---|---|---|
| Temperature | -10°C | | Heating time | 60min | Holding time | 1250min | Vacuum degree | 20pa |

| Secondary drying | | | | | | | | |
|---|---|---|---|---|---|---|---|---|
| Temperature | 0°C | | Heating time | 30min | Holding time | 1250min | Vacuum degree | 20pa |
| Temperature | 25°C | | Heating time | 30min | Holding time | 1250min | Vacuum degree | 20Pa |

The samples after freeze-drying were used for porosity testing.

e. Porosity testing instrument information: Mercury porosimeter, Micromeritics, Autopore 9620;
f. Porosity testing experimental procedure: The sample to be tested was loaded into a penetrometer, and then the penetrometer was inserted into the instrument for mercury injection. The results will provide pore volume distribution for pores with diameters ranging from 360 µm to 0.003 µm.

### Incremental Intrusion (mL/g) Calculation Method:

The incremental intrusion (mL/g) values corresponding to the pore size diameter (nm) range of 6000-9000 were selected and summed to obtain the result.

The term "incremental intrusion" refers to the amount of mercury that enters the pore space of a sample as pressure increases during mercury porosimetry testing. It is a method for measuring how mercury fills pore spaces when pressure is applied, providing results on the internal pore structure and distribution of the sample.

### Experimental results:

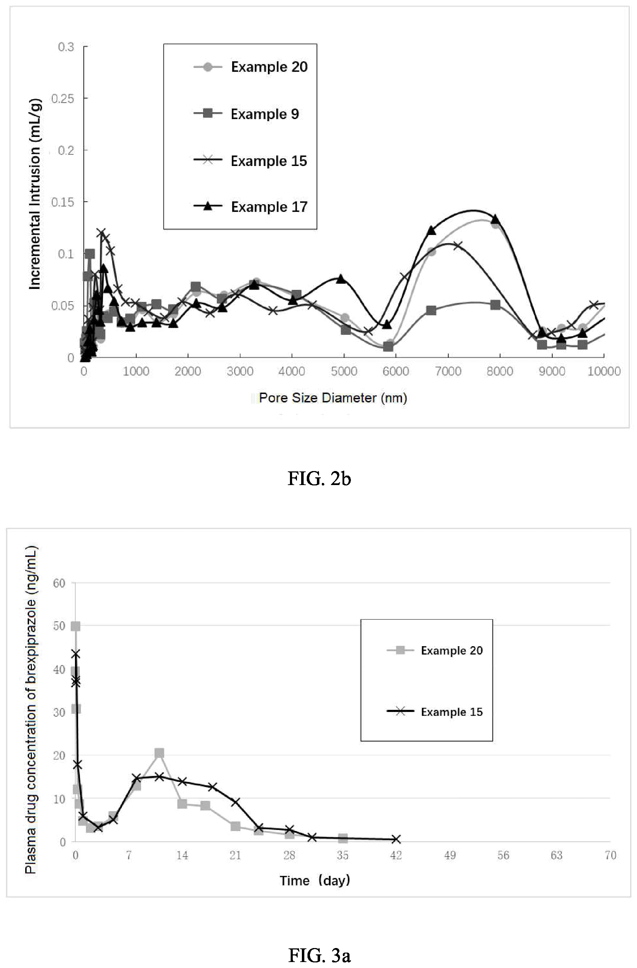

The pore size distribution test results are shown in Table 5 and FIGS. 2a and 2b.

The pore size distribution results showed that in the gel-solidified samples with pore sizes of 6000-9000 nm, the incremental intrusion in internal pore volume was positively correlated with the sum of three times the concentration of brexpiprazole or its pharmaceutically acceptable salt and the contents of the sustained-release material, as well as with the burst release peak and Cmax in rats.

**Table 5**

| Example | API dosage /mg | NMP dosage /mg | PLGA dosage /mg | API content mg/g | API content *3 +PLGA content mg/g | PLGA content / API content | Incremental invasion of the internal pore size distribution ranging from 6000 to 9000 nm after gel dosage form solidification |
|---|---|---|---|---|---|---|---|
| 9 | 240 | 2480 | 560 | 73.2 | 390.2 | 2.33 | 0.1074 |
| 15 | 360 | 3900 | 720 | 72.3 | 361.4 | 2.00 | 0.2067 |
| 17 | 240 | 2800 | 560 | 66.7 | 355.6 | 2.33 | 0.2805 |
| 20 | 60 | 750 | 140. | 63.2 | 336.8 | 2.33 | 0.2564 |

The comparison of Examples 9, 17, and 20 showed that the pore size distribution after gel dosage form solidifying is affected not only by the sum of contents (API content * 3 + PLGA content), but also by the ratio of PLGA content to API content. When the ratio of PLGA content to API content is the same, the general trend is that the lower the sum of contents, the greater the incremental invasion. By simultaneously controlling the sum of API content*3 + PLGA content and the ratio of PLGA content to API content, the desired pore size distribution can be obtained, thereby achieving the desired sustained-release effect.

### Experimental Example 3: Pharmacokinetics Study in Rats

### Experimental methods:

### a. Preparation of gel dosage form samples

Fresh gel dosage form samples were prepared according to the component content ratios in Examples 15 and 20, using the same preparation method as in the above examples, with the following amounts of each component:
Corresponding to the proportions in Example 15, the specific amounts
of each component are: API 360mg, PLGA 720mg, and NMP 3900mg;
Corresponding to the proportions in Example 20, the specific amounts of each component are: API 300mg, PLGA 700mg, and NMP 3750mg.

The gel dosage form samples were sterilized by 25 kGy Gamma irradiation. The final product was obtained after sterilization. Before use, the gel dosage form sample was drawn into a 1mL syringe and kept for later use.

### b. Subcutaneous injection in rats

Six healthy male SD rats (purchased from Zhuhai Bes Test Bio-Tech Co., Ltd.) weighing approximately 200-300g were selected. All rats were housed under the environment of 20-25°C and 40-70% RH, fed with standard feed (SPF rat and mouse maintenance feed, Shenyang Maohua Biotechnology Co., Ltd.), and allowed free access to water and food. Rats were randomly divided into two groups of three each, corresponding to the gel dosage form administration groups in Examples 15 and 20, respectively. A single subcutaneous injection of gel dosage form of brexpiprazole was administered to the lateral buttocks of each test rat, at a dose corresponding to 18 mg/kg of brexpiprazole.

### Orbital blood collection time points in rats:

The blood collection time points for Example 15 were: 0.5 hours, 2 hours, 4 hours, 8 hours, 24 hours, 3 days, 5 days, 8 days, 11 days, 14 days, 18 days, 21 days, 24 days, 28 days, 35 days, and 42 days after medicine administration.

The blood collection time points for Example 20 were: 0.5 hours, 2 hours, 4 hours, 8 hours, 12 hours, 24 hours, 2 days, 3 days, 5 days, 8 days, 11 days, 14 days, 17 days, 21 days, 24 days, 28 days, 31 days, 35 days, and 39 days after medicine administration.

### c. Instruments and testing procedures:

The concentration of brexpiprazole in rat plasma was determined by high performance liquid chromatography-triple quadrupole mass spectrometry (HPLC-MS/MS). The linear range was 0.05 ng/mL to 150 ng/mL. Rat plasma samples were 50 µL. Aripiprazole was used as an internal standard. The samples were pretreated using a protein precipitation method. Then, the responses for brexpiprazole and its internal standard (aripiprazole) were detected under electrospray (+) ion source conditions to compare the differences in pharmacokinetic characteristics of different formulations.

### d. Written description of PK curve plotting:

The obtained PK data were used to plot a PK curve with time on the x-axis and cumulative plasma drug concentration on the y-axis.

### Experimental results:

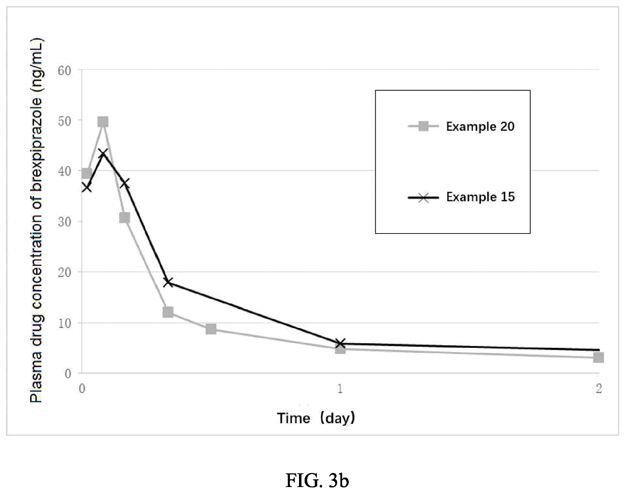

The PK results are shown in Table 6 and FIGS. 3a and 3b. The results showed that the gel dosage form of Example 15 had a better sustained-release effect than that of Example 20, resulting in a brexpiprazole release duration of at least about one month.

**Table 6 PK Parameter Table**

| Item | Example 15 | | Example 20 | |
|---|---|---|---|---|
| | Burst release peak | Second peak | Burst release peak | Second peak |
| Time to peak | 2 hours | 11 days | 2 hours | 11 days |
| Plasma drug concentration (ng/mL) | 43.42 | 15.04 | 49.66 | 20.37 |
| AUC₀₋ₜ (day*ng/mL) | 290.00 | | 221.90 | |
| AUC_{inf} (day*ng/mL) | 290.00 | | 231.87 | |
| T_{1/2} (day) | 9.6 | | 5.2 | |

Note: t is 42 days in Example 15; t is 39 days in Example 20.

The term "burst release peak plasma drug concentration" refers to the first peak plasma drug concentration rapidly achieved by the medicine within a short period after administration.

The term "second peak plasma drug concentration" refers to a relatively high peak plasma drug concentration achieved again after sustained release of the medicine.

The term "AUC₀₋ₜ" refers to the area under the plasma drug concentration-time curve from time 0 to time t, where t refers to the sampling time of the last measurable concentration.

The term "AUC_{inf}" refers to the area under the plasma drug concentration-time curve from 0 to infinity.

The term "T_{1/2}" refers to the time required for the plasma drug concentration to decrease by half.

In this disclosure, relational terms such as first and second are used merely to distinguish one entity or operation from another entity or operation, and do not necessarily require or imply any such actual relationship or order between these entities or operations.

As can be understood from the foregoing, although specific embodiments of this disclosure have been described for illustrative purposes, various modifications or improvements can be made by those skilled in the art without departing from the spirit and scope of this disclosure. These variations or modifications should all fall within the scope of the claims appended to this disclosure.

## Claims

1. A gel dosage form of brexpiprazole, comprising brexpiprazole or a pharmaceutically acceptable salt thereof, at least one biocompatible and biodegradable sustained-release material, and at least one solvent, wherein the concentration of the brexpiprazole or the pharmaceutically acceptable salt thereof is not less than 80 mg/mL.

2. The gel dosage form according to claim 1, wherein the sum of three times the concentration of the brexpiprazole or the pharmaceutically acceptable salt thereof and the concentration of the sustained-release material is not more than 600 mg/mL.

3. The gel dosage form according to claim 1 or 2, wherein the gel dosage form is a clear solution at room temperature.

4. A method for use as an adjunctive therapy to the treatment of major depressive disorder in adults, comprising administering to a patient in need of the method an effective amount of the gel dosage form according to any one of claims 1 to 3.

5. A method for treating schizophrenia, comprising administering to a patient in need of the method an effective amount of the gel dosage form according to any one of claims 1 to 3.

6. A method for treating agitation associated with dementia due to Alzheimer's disease, comprising administering to a patient in need of the method an effective amount of the gel dosage form according to any one of claims 1 to 3.

7. A gel dosage form of brexpiprazole, comprising brexpiprazole or a pharmaceutically acceptable salt thereof, at least one biocompatible and biodegradable sustained-release material, and at least one solvent, wherein, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not more than 500 mg/g, preferably not more than 455 mg/g, more preferably not more than 441 mg/g, or further preferably not more than 400 mg/g.

8. The gel dosage form according to claim 7, wherein the content of the brexpiprazole or the pharmaceutically acceptable salt thereof is not less than 28 mg/g, optionally, the content of the brexpiprazole or the pharmaceutically acceptable salt thereof is in a range of 28 mg/g to 102 mg/g, optionally 66 mg/g to 73 mg/g.

9. The gel dosage form according to claim 7 or 8, wherein, based on the total mass of the brexpiprazole or the pharmaceutically acceptable salt thereof, the sustained-release material and the solvent, the sum of three times the content of the brexpiprazole or the pharmaceutically acceptable salt thereof and the content of the sustained-release material is not less than 322 mg/g.

10. The gel dosage form according to any one of claims 7 to 9, wherein the sum of three times the content of the brexpiprazole or the pharmaceutically acceptable salt thereof and the content of the sustained-release material is 361 mg/g to 391 mg/g, preferably 371 mg/g to 391 mg/g, and more preferably 371 mg/g to 388 mg/g.

11. The gel dosage form according to claim 10, wherein, based on the total mass of the brexpiprazole or the pharmaceutically acceptable salt thereof, the sustained-release material and the solvent, the ratio of the content of the sustained-release material to the content of the brexpiprazole or the pharmaceutically acceptable salt thereof is (0.8-10):1, preferably (0.8:-3.3):1, and more preferably (2.0-2.3):1.

12. Use of the gel dosage form according to any one of claims 7 to 11 in the manufacture of a medicine for use as an adjunctive therapy to the treatment of major depressive disorder in adults.

13. Use of the gel dosage form according to any one of claims 7 to 11 in the manufacture of a medicine for treating schizophrenia.

14. Use of the gel dosage form according to any one of claims 7 to 11 in the manufacture of a medicine for treating agitation associated with dementia due to Alzheimer's disease.

15. A method for use as an adjunctive therapy to the treatment of major depressive disorder in adults, comprising administering to a patient in need of the method an effective amount of the gel dosage form according to any one of claims 7 to 11.

16. A method for treating schizophrenia, comprising administering to a patient in need of the method an effective amount of the gel dosage form according to any one of claims 7 to 11.

17. A method for treating agitation associated with dementia due to Alzheimer's disease, comprising administering to a patient in need of the method an effective amount of the gel dosage form according to any one of claims 7 to 11.
